# EUROPÄISCHE PATENTANMELDUNG

(11) **EP 4 268 867 A1**
(43) Veröffentlichungstag der Anmeldung: **01.11.2023**
(21) Anmeldenummer: 22170085.9
(22) Anmeldetag: 26.04.2022
(51) Int. Cl.: A61M 11/00, A61M 15/00, A61M 15/08, B05B 11/00, G16H 20/13

(54) **SPENDER UND AUSWERTEEINHEIT HIERFÜR**

(71) Anmelder: Aptar Radolfzell GmbH, 78315 Radolfzell (DE)
(72) Erfinder: Helmlinger, Michael, 78315 Radolfzell-Böhringen (DE); Kohnle, Jörg, 78056 Villingen-Schwenningen (DE)
(74) Vertreter: Witte, Weller und Partner Patentanwälte mbB Stuttgart

(57) **Zusammenfassung**

Bekannt ist ein Spender (10) zum Austrag pharmazeutischer oder kosmetischer Medien mit einem Gehäuse (12), einem Medienspeicher (14) sowie einer Austragöffnung (16). Bekannt ist es weiterhin, einen solchen Spender mit einer elektronischen Auswerteeinheit (40) zur Erfassung der Nutzung des Spenders zu versehen, die zur lösbaren Anbringung am Gehäuse (12) des Spenders (10) ausgebildet ist.

Es wird vorgeschlagen, dass die elektronische Auswerteeinheit (40) eine Anlageseite (42) aufweist, die im angebrachten Zustand der Auswerteeinheit (40) in Richtung des Gehäuses (12) weist. Die elektronische Auswerteeinheit (40) weist an der Anlageseite (42) zwei zueinander parallele Anlagerippen (44) zur Anlage am Gehäuse (12) auf, insbesondere zur Anlage an einem Speicherkörper (18) des Medienspeichers (14).

## Beschreibung

### ANWENDUNGSGEBIET UND STAND DER TECHNIK

Die Erfindung betrifft einen Spender zum Austrag pharmazeutischer oder kosmetischer Medien sowie insbesondere eine elektronische Auswerteeinheit für einen solchen Spender.

Ein gattungsgemäßer Spender dient dem Austrag von Medien, insbesondere von pharmazeutischen Flüssigkeiten. Es ist bekannt, solche Spender mit Auswerteeinheiten zu versehen, die über eine Sensorik die Nutzung des Spenders erfassen, beispielsweise um eine nachfolgende Prüfung zu gestatten, ob ein Medikament in richtiger Weise und in Übereinstimmung mit einem Medikationsplan eingenommen wird.

Bekannte Auswerteeinheiten sind in den jeweiligen Spender baulich integriert oder sind lösbar am Spender angebracht, so dass sie vom Spender gelöst und an einen anderen Spender gleichen Typs angebracht werden können.

Aufgrund vieler unterschiedlicher Spendertypen ist der Aufwand zur jeweiligen Ergänzung der Spender um Auswerteeinheiten erheblich.

### AUFGABE UND LÖSUNG

Aufgabe der Erfindung ist es, eine Auswerteeinheit für einen Spender zur Verfügung zu stellen, die es gestattet, mit Spendern unterschiedlicher Art verwendet zu werden und sicher an diesen unterschiedlichen Spendern zum Zwecke der Erfassung der Nutzung befestigt zu werden.

Erfindungsgemäß wird ein Spender zum Austrag pharmazeutischer oder kosmetischer Medien vorgeschlagen, der über eine elektronische Auswerteeinheit verfügt. Die Erfindung betrifft darüber hinaus auch die Auswerteeinheit als solche.

Ein erfindungsgemäßer Spender weist ein Gehäuse auf, das die Außenkontur des Spenders bildet und mehrteilig ausgebildet sein kann. Insbesondere kann ein solches Gehäuse einen Speicherkörper aufweisen, der den Medienspeicher und insbesondere einen Flüssigkeitsspeicher bildet, in dem das Medium, insbesondere eine pharmazeutische Flüssigkeit, vor dem Austrag gelagert ist.

Der Spender weist eine Austragöffnung auf, durch die Medium aus dem Medienspeicher in eine umgebende Atmosphäre abgegeben werden kann.

Bevorzugt ist der Spender zur Ausbringung von Flüssigkeit ausgebildet, insbesondere in Form eines Strahls, eines Sprühstrahls, oder in Form von Einzeltropfen. Stattdessen kann der Spender jedoch auch für andere Medien vorgesehen sein, so insbesondere zur Ausbringung von Pulver zum Zwecke der Inhalation.

Ist der Spender als Flüssigkeitsspender ausgebildet, so kann es sich insbesondere um einen Pumpspender handeln, der über eine manuell betätigbare Pumpeinrichtung verfügt, die Flüssigkeit aus dem Flüssigkeitsspeicher zur Austragöffnung fördert. Alternativ handelt es sich um einen Quetschflaschenspender, der einen elastisch verformbaren Speicherkörper aufweist, durch dessen manuelle Komprimierung Flüssigkeit ausgetragen werden kann.

Der Spender kann weiterhin als Inhalator ausgebildet sein, insbesondere als Spender mit einem zur Abgabe einer Dosis der Flüssigkeit niederdrückbarem Container zur Abgabe einer definierten Dosis des Medikaments. Gemäß einem weiteren Beispiel kann der Spender als Nasalspender ausgebildet sein, insbesondere mit einem langgestreckten Nasalapplikator, an dessen distalem Ende die Austragöffnung vorgesehen ist.

Die elektronische Auswerteeinheit ist zur Erfassung der Nutzung des Spenders ausgebildet. Sie weist hierfür elektrische bzw. elektronische Komponenten auf, insbesondere eine Batterie, einen Prozessor und mindestens einen Sensor. Über den Sensor werden Daten erfasst, die Rückschlüsse auf die Spendernutzung zulassen. Die Sensoren können dabei dafür ausgebildet sein, unmittelbar eine externe Kraftbeaufschlagung zu erfassen, wie sie beispielsweise beim Niederdrücken einer Betätigungshandhabe zur Durchführung einer Pumpbetätigung auftritt. Vorzugsweise ist jedoch mindestens ein Sensor vorgesehen, der berührungslos und ohne externe Kraftbeaufschlagung Daten erfassen kann, wobei es sich insbesondere um ein Mikrofon und/oder einen Bewegungssensor handeln kann. Diese Arten von Sensoren eigenen sich besonders gut dafür, an Spendern unterschiedlicher Art und Ausbringungsart verwendet zu werden, wobei eine spendertypspezifische Anpassung bezüglich der Hardware nicht erforderlich ist. Beispielsweise können die Ausgangsdaten eines Mikrofons ausgewertet werden, um die beim jeweiligen Spendertyp charakteristischen Geräusche während der Nutzung zu erkennen.

Die Auswerteeinheit umfasst vorzugsweise eine Ausgabeeinrichtung, insbesondere in Form mindestens einer LED, eines Displays, eines Vibrators und/oder eines Lautsprechers, um Signale an den Nutzer abzugeben, beispielsweise um die Information zu übermitteln, dass ein Austrag sensiert worden ist oder gemäß Medikationsplan ansteht.

Die Auswerteeinheit ist vorzugsweise dafür ausgebildet, zusammen mit einem externen Gerät wie insbesondere einem Smartphone verwendet zu werden. Zur Kommunikation mit dem externen Gerät weist die Auswerteeinheit vorzugsweise ein Funkmodul, insbesondere ein Bluetooth- und/oder WIFI-Funkmodul oder aber ein GSM-/3G-/4G- oder 5G-Funkmodel auf.

Die Auswerteeinheit ist zur lösbaren Anbringung am Gehäuse des Spenders und insbesondere am genannten Speicherkörper des Gehäuses vorgesehen. Um eine sichere Anlage der Auswerteeinheit am Gehäuse zu ermöglichen, weist die elektronische Auswerteeinheit eine Anlageseite auf, die im angebrachten Zustand der Auswerteeinheit in Richtung des Gehäuses des Spenders weist, wobei an dieser Anlageseite mindestens zwei zueinander parallele Anlagerippen zur Anlage am Gehäuse vorgesehen sind. Vorzugsweise handelt es sich um genau zwei oder drei zueinander parallele Anlagerippen, wobei die einzelnen Anlagerippen auch unterbrochen sein können und aus zueinander fluchtenden Rippensegmenten bestehen kann. Die Rippen bilden erhabene Bereiche zur Anlage am Gehäuse. Die Rippen weisen vorzugsweise eine Länge zwischen 10 mm und 30 mm auf. Die Breite der Rippen beträgt vorzugsweise 5 mm oder weniger, insbesondere vorzugsweise 3 mm oder weniger. An ihrer zum Gehäuse weisenden Seite sind die Rippen vorzugsweise mit einer ausgerundeten Kante versehen.

Die im montierten Zustand bestimmungsgemäß am Spendergehäuse anliegenden Anlagerippen sind vorzugsweise an einer Rückseite eines Gehäuses der Auswerteeinheit vorgesehen.

Zwischen den mindestens zwei Anlagerippen ist ein zurückgesetzter Zwischenbereich vorgesehen, der die Anlagerippen voneinander trennt. In diesem Zwischenbereich ist die Anlageseite der Auswerteeinheit derart zurückgesetzt, so dass sie nicht am Gehäuse des Spenders anliegt. Der Kontakt zwischen der Anlageseite und dem Gehäuse des Spenders beschränkt sich demnach auf die mindestens zwei Anlagerippen.

Eine Auswerteeinheit mit solchen Anlagerippen gestattet eine sichere Anlage am Gehäuse des Spenders, da die Andruckkraft in definierten Anlagebereichen stattfindet. Zudem gestatten die Anlagerippen die Anlage an Gehäusen unterschiedliche Maße und insbesondere Speicherkörper mit unterschiedlich gewölbter Außenkontur.

Welche Krümmungsradien solcher Außenkonturen jeweils abgedeckt werden, hängt von der Geometrie der Anlageseite ab. Bevorzugt ist eine Gestaltung, bei der die Anlagerippen einen Abstand voneinander von mindestens 8 mm aufweisen, wobei sich die Anlagerippen vorzugsweise um mindestens 1 mm, vorzugsweise um mindestens 1,5 mm über die Anlagefläche in diesem Zwischenbereich erheben. Zwei Anlagerippen, zwischen denen ein Zwischenbereich von 8 mm vorgesehen ist, der 1 mm zurückgesetzt ist, können an Gehäuseabschnitten mit einem Krümmungsradius größer 10 mm angebracht werden, ohne dass der Gehäuseabschnitt abseits der Anlagerippen in Kontakt mit der Anlageseite gelangt. Mit einem Zwischenbereich von 8 mm Weite, der 1,5 mm zurückgesetzt ist, ist die Anbringung bis zu einem Krümmungsradius von ca. 6 mm möglich.

Eine oder mehrere der Anlagerippen können an einem elastischen Ausleger angebracht sein, insbesondere an einem Ausleger in Form einer an drei Seiten freigeschnittenen Kunststoffzunge, die ein federndes Verhalten orthogonal zur Anlageseite ermöglicht. Es kann auch vorgesehen sein, dass eine oder mehrere, nicht aber alle Anlagerippen derartig auslenkbarsind, während mindestens eine Anlagerippe fest und nicht auslenkbar an der Anlageseite der Auswerteeinheit vorgesehen ist. Eine mögliche Konstellation sieht beispielsweise drei Anlagerippen vor, von denen eine oder zwei an jeweils einem elastischen Ausleger angebracht sind, während die dritte Anlagerippe ortsfest an der Anlageseite vorgesehen ist. Auch eine Rippengestaltung mit zwei Anlagerippen, die beide auslenkbar sind, kann zweckmäßig sein.

Im Falle einer Anlagerippe, die aus mehreren zueinander fluchtenden Rippensegmenten besteht, kann es vorteilhaft sein, wenn diese Rippensegmente an separaten elastischen Auslegern vorgesehen sind.

Die Anlagerippen als solche reichen nicht aus, um die Auswerteeinheit am Gehäuse des Spenders zu befestigen, sondern sie bewirken lediglich das sichere Anliegen der Auswerteeinheit an der Außenseite des Gehäuses.

Zum Zwecke der dortigen Befestigung sind unterschiedliche Konzepte möglich.

Bei einer bevorzugten Art der Gestaltung ist vorgesehen, dass die elektronische Auswerteeinheit ein formflexibles Befestigungsband aufweist, welches an der Auswerteeinheit angebracht ist und das Gehäuse umgibt, insbesondere den Speicherkörper. Mittels dieses Befestigungsbandes wird die Auswerteeinheit an das Gehäuse angedrückt. Zusammen mit den Anlagerippen lässt sich so ein sicherer Halt erzielen.

Das Befestigungsband kann als elastisches Band oder auch als weitgehend unelastisches Band ausgestaltet sein. Insbesondere bei Verwendung eines unelastischen Bandes ist es zweckmäßig, mindestens eine Anlagerippe an einem elastisch auslenkbaren Ausleger vorzusehen.

Ist das Befestigungsband elastisch, so kann es ohne Längenanpassung für Gehäuse und insbesondere Speicherkörper unterschiedlicher Umfänge verwendet werden.

Das Befestigungsband kann auf verschiedene Weisen an einem Gehäuse der Auswerteeinheit befestigt sein. Eine bevorzugte Gestaltung sieht vor, dass das Befestigungsband umlaufend geschlossen wird und zweisträngig um das Gehäuse herumgelegt wird, wobei die beiden jeweilig endseitigen Schlaufen am Gehäuse befestigt sind oder werden. Durch die Schlaufen ist die Befestigung am Gehäuse der Auswerteeinheit einfach möglich. Ein Befestigungsband, das derartig zweisträngig um das Gehäuse des Spenders herumgelegt wird, weist vorzugsweise einen kreisrunden Querschnitt auf und kann durch einen einfachen O-Ring gebildet werden.

Auch ist es möglich, das Befestigungsband als geschlossenes Band auszugestalten, welches das Gehäuse des Spenders vollständig umgibt. Das Band kann bei einer solchen Gestaltung insbesondere in einem umfänglich geschlossenen und durchgehenden Schacht des Gehäuses der Auswerte einheit angeordnet sein und so sicher an der Auswerteeinheit befestigt sein.

Insbesondere bei einem wenig elastischen Band kann es zweckmäßig sein, dass das Befestigungsband zwei Bandabschnitte umfasst, die jeweils an einer Haupteinheit oder einem Gehäuse der Auswerteeinheit befestigt sind. Die beiden Bandabschnitte sind im Bereich eines Verschlusses wie beispielsweise eines Magnet- oder Klettverschlusses miteinander verbindbar, um die Auswerteinheit hierdurch am Gehäuse des Spenders zu befestigen. Das Befestigungsband kann auch als längenveränderliches Band ausgebildet sein, welches mittels eines Verschlusses in einer gewünschten Längenkonfiguration festgestellt werden kann. Es kann zweckmäßig sein, wenn der Verschluss zusammenwirkende Verschlusselemente an beiden Bandabschnitten aufweist, wobei an einem der Bandabschnitte vorzugsweise eine Mehrzahl von Verschlusselementen vorgesehen sind, so dass je nach Umfang des Gehäuses jeweils ein passendes Verschlusselement zum Zusammenfügen der Bandabschnitte gewählt werden kann.

Alternativ oder zusätzlich zum Band kann auch an der Außenseite des Gehäuses des Spenders, insbesondere an dessen Speicherkörper, eine magnetische oder magnetisierbare Haltefläche vorgesehen sein. Dies gestattet es, im Bereich dieser Haltefläche die Auswerteeinheit anzulegen, wobei die Anlageseite der Auswerteeinheit hierfür korrespondierend zur gehäuseseitigen Haltefläche magnetisch oder magnetisierbar ausgebildet ist.

Vorzugsweise ist am Gehäuse eine magnetisierbare und insbesondere eine metallische Haltefläche vorgesehen, während an der Auswerteeinheit mindestens ein Permanentmagnet vorgesehen ist, aber auch die umgekehrte Konstellation ist möglich. Auch ist eine Gestaltung möglich, bei der sowohl die Haltefläche als auch magnetische Gestaltung der Auswerteeinheit mit Permanentmagneten realisiert ist.

Als Permanentmagneten kommen insbesondere Neodym-Magneten in Frage, da solche Magneten eine starke Magnetkraft bereitstellen und daher für die sichere Befestigung der Auswerteeinheit am Gehäuse des Spenders besonders von Vorteil sind.

Die am Gehäuse vorgesehene Haltefläche kann auf verschiedene Arten bereitgestellt werden. Insbesondere bevorzugt ist es, wenn die Haltefläche im Bereich eines beschrifteten Etiketts vorgesehen ist. Sie kann unmittelbar das Etikett bilden oder mittels des Etiketts an einer Gehäusefläche des Gehäuses festgeklebt sein. Die Haltefläche kann auch unabhängig vom Etikett selbstständig am Gehäuse mittels einer Klebeverbindung angebracht sein. Auch sind Gestaltungen möglich, bei denen die Haltefläche durch eine Haltehülse oder Halteklemme gebildet wird, die klemmend am Gehäuse des Spenders angebracht ist.

Eine weitere Möglichkeit der Befestigung der Auswerteeinheit am Gehäuse sieht vor, dass die Auswerteeinheit mindestens zwei und vorzugsweise mindestens drei elastisch auslenkbare Haltearme aufweist, die das Gehäuse, insbesondere einen Speicherkörper des Gehäuses, umgreifen und damit einen klemmenden Halt der Auswerteinheit am Gehäuse bewirken. Vorzugsweise sind mindestens drei Haltearme vorgesehen, von denen zwei Haltearme sich in einer ersten Erstreckungsrichtung erstrecken und von denen der dritte Haltearm zwischen diesen beiden Haltearmen angeordnet ist und sich in entgegengesetzter Erstreckungsrichtung erstreckt.

Die Auswerteeinheit kann ein Gehäuse aufweisen, welches nicht-modular aufgebaut ist und für den Anwender nicht weiter zerlegbar. Eine besondere Ausgestaltung eines erfindungsgemäßen Spenders und einer erfindungsgemäßen Auswerteeinheit sieht jedoch vor, dass die Auswerteeinheit eine Befestigungseinheit und eine Elektronikeinheit umfasst, die getrennte Baueinheiten darstellen.

Die Befestigungseinheit ist dabei zur lösbaren Anbringung am Gehäuse ausgebildet und bildet die Anlageseite der Auswerteeinheit und die daran vorgesehenen Anlagerippen. Die hiervon trennbare Elektronikeinheit ist zur lösbaren Anbringung an der Befestigungseinheit vorgesehen. Die Elektronikeinheit umfasst vorzugsweise die Gesamtheit der elektronischen Komponenten der Auswerteeinheit, mindestens jedoch die Batterie, den Prozessor sowie mindestens einen Sensor.

Die Zweiteilung der Auswerteeinheit gestattet es, die Elektronikeinheit mit unterschiedlichen Befestigungseinheiten zu verwenden, von denen nicht alle in oben beschriebener Weise über Anlagerippen verfügen müssen. Es können jedoch auch unterschiedliche Befestigungseinheiten vorgesehen sein, die jeweils über Anlagerippen verfügen, wobei diese unterschiedlich ausgestaltet und insbesondere beabstandet sind, so dass die Befestigungseinheiten jeweils für Spendergehäuse unterschiedlicher Formgebung geeignet sind.

Die gleiche Elektronikeinheit kann demnach durch Konfiguration mit unterschiedlichen Befestigungseinheiten zur Anbringung an vielen unterschiedlichen Spendern vorgesehen sein.

Die Möglichkeit, die Befestigungseinheit und die Elektronikeinheit zu trennen und in anderer Konfiguration wieder zusammenzusetzen, muss nicht zwingend für den Endanwender gegeben sein. Die genannte Modularität kann auch dafür genutzt werden, um einheitliche Elektronikeinheiten bereits im Rahmen der Herstellung und Bereitstellung bedarfsgerecht mit passenden Befestigungseinheiten zu versehen und ggf. fest zu verbinden.

Besonders bevorzugt ist es allerdings, wenn die Befestigungseinheit zur werkzeuglos lösbaren Anbringung an der Elektronikeinheit ausgebildet ist und damit dem Endanwender selbst die Möglichkeit des Wechsels der Befestigungseinheit offensteht. Insbesondere kann zu diesem Zweck vorgesehen sein, dass die Elektronikeinheit magnetisch an der Befestigungseinheit befestigt ist oder die Elektronikeinheit durch einen Rastmechanismus mit einer elastisch auslenkbaren Rastlasche an der Befestigungseinheit befestigt ist.

Die Befestigungseinheit ist vorzugsweise frei von elektronischen Komponenten. Sie kann jedoch auch Träger von Sensoren sein und/oder ein elektronisch auslesbares Identifikationsmerkmal aufweisen, beispielsweise ein Identifikationsmerkmal, das in einem RFID-Chip der Befestigungseinheit abgelegt ist und durch einen Sensor der Elektronikeinheit auslesbar ist. Hierdurch kann protokolliert werden, mit welcher Befestigungseinheit die Elektronikeinheit verbunden ist.

Die erfindungsgemäße Auswerteeinheit kann im Verkauf zusammen mit dem Spender als Set gehandhabt werden. Die Auswerteinheit kann aber auch separat erworben werden, um sie mit bestehenden Spendern zu verwenden. Von Vorteil kann es daher sein, wenn die elektronische Auswerteeinheit eine Mehrzahl von Befestigungsbändern umfasst, die wahlfrei an der Auswerteinheit, insbesondere an der Befestigungseinheit, anbringbar sind. Auch ist es möglich, dass die Auswerteeinheit über eine Mehrzahl von Befestigungseinheiten verfügt, die wahlfrei an der Elektronikeinheit angebracht werden können, um die Auswerteeinheit zur Anbringung an einem bestimmten Spendertyp zu konfigurieren.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig.1 bis 3 zeigen exemplarisch einen erfindungsgemäßen Flüssigkeitsspender mit einer erfindungsgemäßen Auswerteeinheit.
Fig. 4 bis 6 verdeutlichen eine erste Konfiguration von Anlagerippen an einer Anlageseite der Auswerteeinheit.
Fig. 7 bis 8 verdeutlichen eine zweite Konfiguration von Anlagerippen an einer Anlageseite der Auswerteeinheit.
Fig. 10 bis 14 zeigen verschiedene Ausgestaltungen von Befestigungsbändern zur Anbringung der Auswerteeinheit am Gehäuse des Spenders.
Fig. 15 bis 17 zeigen die magnetische Anbringung der Auswerteeinheit am Gehäuse des Spenders.
Fig. 18 bis 21 zeigen eine mögliche modulare Ausgestaltung der Auswerteeinheit mit einer Befestigungseinheit und einer Elektronikeinheit sowie Kopplungsmittel zur Kopplung der Elektronikeinheit an die Befestigungseinheit.

### DETAILLIERTE BESCHREIBUNG DER AUSFÜHRUNGSBEISPIELE

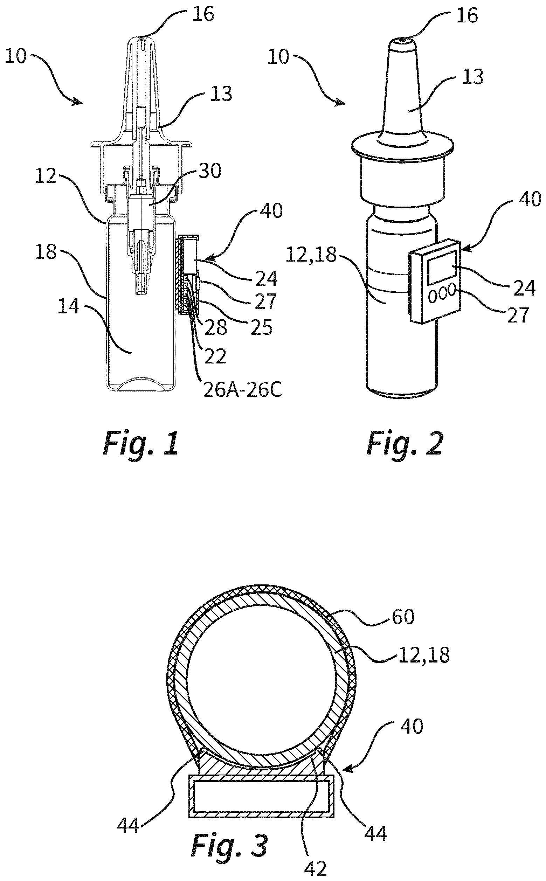

Fig. 1 bis 3 zeigen einen erfindungsgemäßen Spender 10, der zum Austrag pharmazeutischer Flüssigkeiten ausgebildet ist. Der Flüssigkeitsspender 10 ist exemplarisch als Nasalspender ausgebildet und verfügt über ein Applikatorteil mit einem länglichen Nasalapplikator 13, an dessen distalem Ende eine Austragöffnung 16 vorgesehen ist. Der konkrete Spendertyp ist jedoch nur als Beispiel zu verstehen. Die Erfindung umfasst ebenso auch andere Spenderfür Medien und insbesondere Flüssigkeiten, insbesondere pharmazeutische Spender, die zur oralen oder topischen Abgabe von Medikamenten ausgebildet sind.

Durch Niederdrücken des Applikatorteils gegenüber einem Flüssigkeitsspeicher 14 bzw. dem den Flüssigkeitsspeicher bildenden Speicherkörper 18 kann eine Pumpeinrichtung 30 betätigt werden, die Flüssigkeit aus dem Flüssigkeitsspeicher 14 zur Austragöffnung 16 fördert.

Bei einem erfindungsgemäßen Spender 10 ist vorgesehen, dass die Nutzung des Spenders 10 durch den Anwender elektronisch erfasst wird. Hierfür ist eine elektronische Auswerteeinheit 40 vorgesehen. Diese umfasst im vorliegenden Beispiel eine Batterie 25, eine Anzeigeeinrichtung 24, eine Platine mit einem Prozessor 28, Bedienelemente 27 sowie verschiedene Sensoren 26A bis 26C.

Die Sensoren 26A-26C können insbesondere einen Bewegungssensor umfassen, der die Ausrichtung des Spenders und/oder Beschleunigungen erfasst. Weiterhin können die Sensoren 26A-26C ein Mikrofon umfassen, welches Geräusche erfasst, die bei der Handhabung des Spenders 10 auftreten. Die Auswertung der erfassten Daten erfolgt entweder direkt mittels des Prozessors 28 oder ganz oder teilweise auf einem externen System, welches über eine Funkschnittstelle mit der Auswerteeinheit 40 verbunden ist.

Exemplarisch kann der Prozessor 28 dafür ausgebildet sein, im Falle von mittels des Bewegungssensors erfassten Bewegungen des Spenders 10 das Mikrofon zu aktivieren. Mittels des Mikrofons können Geräusche erfasst werden, insbesondere durch das Spendergehäuse 12 übertragene Geräusche. Insbesondere gehen die verschiedenen Phasen eines Pumpvorgangs, also das Ausbringen von Flüssigkeit aus einer Pumpkammer und das erneute Ansaugen von Flüssigkeit aus dem Medienspeicher 14 in die Pumpkammer mit charakteristischen Geräuschen einher, die gut voneinander unterscheidbarsind.

Die Auswerteeinheit 40 ist am Speicherkörper 18 des Spendergehäuses 12 angebracht. Der Speicherkörper 18 weist eine zylindrische Grundform auf, vorliegend eine kreiszylindrische Grundform. Die Auswerteeinheit 40 verfügt über ein Befestigungsband 60, welches den Speicherkörper 18 umgibt und mittels dessen die Auswerteeinheit 40 an den Speicherkörper angepresst wird.

Dabei liegt die Auswerteeinheit 40 nicht vollflächig am Speicherkörper 18 an, sondern im Bereich von Anlagerippen 44, die an der Anlageseite 42 der Auswerteeinheit 40 vorgesehen sind. In Fig. 3 ist dies anhand einer Schnittdarstellung exemplarisch verdeutlicht.

Durch die Anlagerippen 44 ist der direkte Anlagekontakt zwischen dem Spendergehäuse 12 und der Auswerteeinheit 44 auf die Anlageflächen der Anlagerippen 44 beschränkt, so dass hier eine vergleichsweise große Flächenpressung und ein sicherer Halt erzielt wird. Insbesondere die Auswertungen von Schallwellen, die durch die Nutzung des Spenders bedingt sind und bestimmungsgemäß durch das Mikrofon erfasst werden, ist durch die Anlagerippen 44 in besserer Weise möglich.

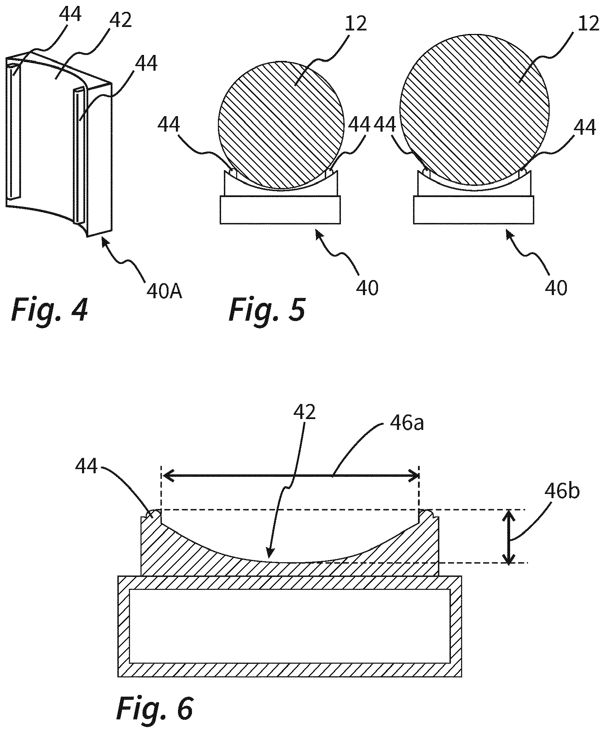

Die Fig. 4 bis 6 zeigen eine erste Konfiguration von Anlagerippen 44. In Fig. 4 ist die Rückseite der Auswerteeinheit 40 dargestellt. Diese Rückseite bildet eine Anlageseite 42, wobei zwei parallele Anlagerippen 44 zueinander ortsfest an der Anlageseite 42 vorgesehen sind. Zwischen den beiden Anlagerippen 44 ist ein zurückgesetzter Zwischenbereich vorgesehen. Wie sich anhand der Fig. 5 ersehen lässt, ermöglichen es die Anlagerippen 44, an Spendergehäusen 12 unterschiedlicher Krümmungsradien anzuliegen. Die gleiche Auswerteeinheit 40 bzw. die gleiche Befestigungseinheit 40A der Auswerteeinheit 40 kann also für unterschiedliche Spender 10 gleichermaßen gut verwendet werden.

Fig. 6 verdeutlicht nochmals die Geometrie der Anlageseite 42. Es ist zu ersehen, dass diese eine grundsätzlich gewölbte konkave Form aufweist und dass die beiden Anlagerippen 44 sich über diese gewölbte konkave Form erheben. Der Abstand 46A entspricht der Breite des Zwischenbereichs. Der Abstand 46B zeigt an, wieweit der Zwischenbereich maximal gegenüber den Anlagerippen 44 zurückgesetzt ist.

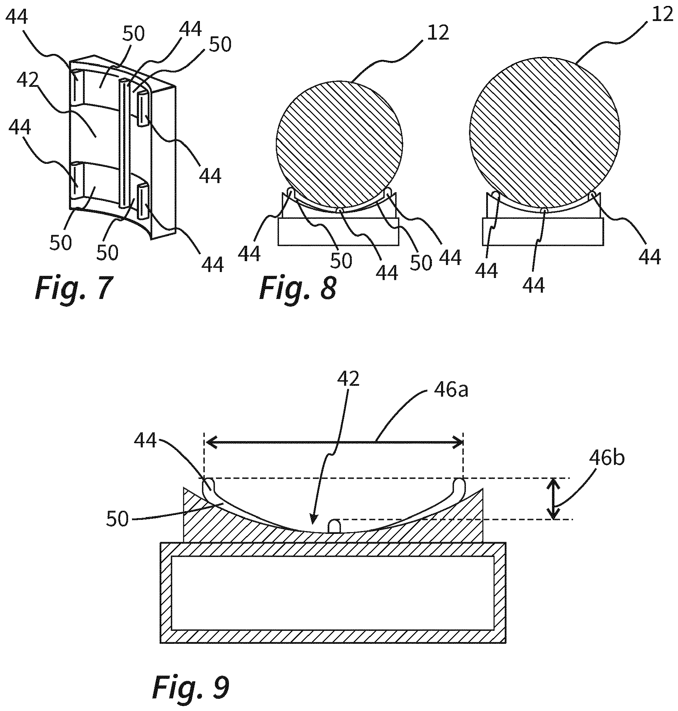

Die Fig. 7 bis 9 zeigen eine zweite Konfiguration von Anlagerippen 44. Wie anhand der Fig. 7 zu ersehen ist, ist die Anlageseite 42 hier mit insgesamt drei Anlagerippen 44 versehen. Eine zentrische Anlagerippe 44 ist fest mit dem Gehäuse der Auswerteeinheit 40 verbunden und gegenüber diesem nicht in relevantem Maße auslenkbar. Zwei seitliche Anlagerippen 44 sind dagegen aus Auslegern 50 vorgesehen, wobei beide seitliche Anlagerippen 44 jeweils aus zwei miteinander fluchtenden Rippensegmenten bestehen, die ihrerseits jeweils an einem Ausleger 50 vorgesehen sind. Die beiden äußeren Anlagerippen können daher federnd ausgelenkt werden.

Die Ausleger 50 gestatten es, die außenliegenden Anlagerippen 44 gegen die Federkraft der Ausleger 50 relativ zueinander zu verlagern. Hierdurch kann trotz insgesamtdreier Anlagerippen erreicht werden, dass alle Anlagerippen bei Verwendung der Auswerteeinheit 40 mit unterschiedlichen Spendergehäusen 12 bzw. Speicherkörpern 18 jeweils am Spendergehäuse 12 anliegen. Bei Spendergehäusen 12/Speicherkörpern 18 mit größeren Durchmessern werden die Ausleger 50 elastisch ausgelenkt, wobei dies insbesondere im Kontext einer Befestigung der Auswerteinheit 40 mit einem Befestigungsband 60 von Vorteil sein kann.

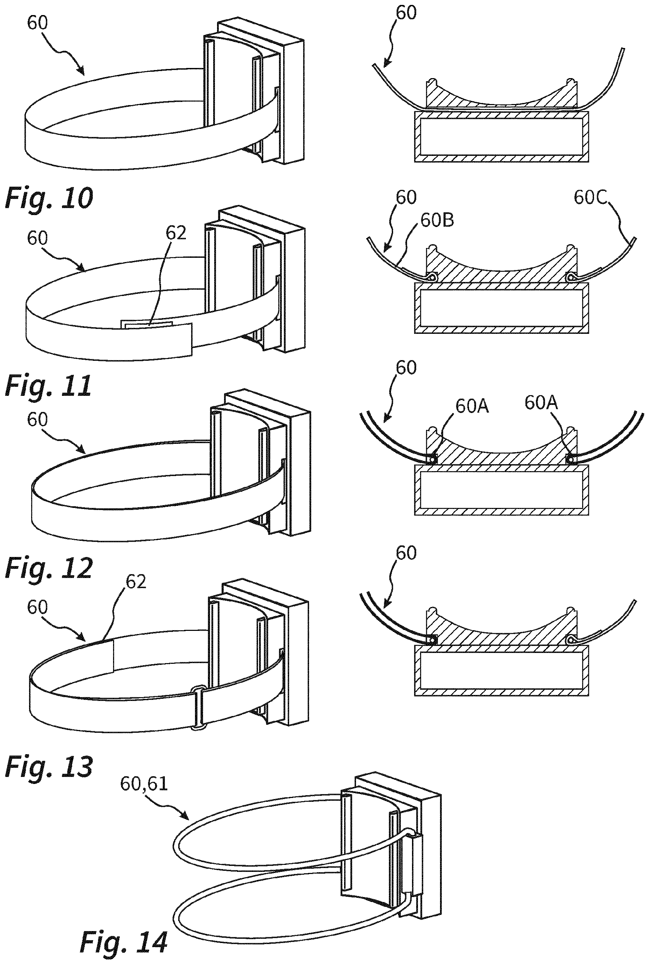

Die Fig. 10 bis 14 zeigen unterschiedliche Konfigurationen eines solchen Befestigungsbandes 60. Die jeweils dargestellten Befestigungsbänder 60 sind an einem Gehäuse der Auswerteeinheit 40 angebracht und werden um das Spendergehäuse 12 herumgelegt.

Bei der Gestaltung der Fig. 10 ist das Befestigungsband 60 als vollständig umlaufendes Befestigungsband 60 gestaltet, welches im Bereich des Gehäuses der Auswerteeinheit 40 durch einen Schacht 64 zwischen einer Elektronikeinheit 40B und der Befestigungseinheit 40A geführt ist. Das Befestigungsband ist elastisch ausgebildet und kann beispielsweise aus Gummi gefertigt sein. Es gestattet die Anbringung an Spendergehäusen unterschiedlichen Umfangs.

Bei der Gestaltung gemäß Fig. 11 weist das Befestigungsband zwei Bandabschnitte 60B, 60C auf, die jeweils am Gehäuse der Auswerteeinheit 40 befestigt sind. Die Bandabschnitte 60B, 60C können von beiden Seiten um das Spendergehäuse 12 herumgelegt werden und mittels eines Verschlusses 62 miteinander verbunden werden. Der Verschluss 62 kann beispielsweise ein Magnetverschluss sein.

Bei der Gestaltung der Fig. 12 findet ein geschlossenes Befestigungsband 60 Verwendung, welches jedoch doppelt um das Spendergehäuse 12 herumgelegt wird und im Bereich des Gehäuses der Auswerteeinheit in Form zweier Schlaufen 60A gelegt ist, die am Gehäuse der Auswerteeinheit 40 befestigt sind.

Die Gestaltung der Fig. 13 sieht vor, dass das dort Verwendung findende Befestigungsband 60 längenveränderlich ausgestaltet ist und in der für das Spendergehäuse richtigen Einstellung mittels eines Verschlusses 62 gesichert werden kann.

Die Gestaltung der Fig. 14 ähnelt jener der Fig. 12. Auch hier findet ein geschlossenes Befestigungsband 60 Verwendung, welches zweisträngig um das Spendergehäuse 12 herumgelegt wird und welches im Bereich seiner Enden zwei Schlaufen ausbildet, die am Gehäuse der Auswerteeinheit 40 befestigt sind. Abweichend von der Gestaltung der Fig. 12, bei der ein flaches Befestigungsband 60 genutzt wird, ist das Befestigungsband 60 der Fig. 14 als O-Ring mit kreisrundem Querschnitt gebildet.

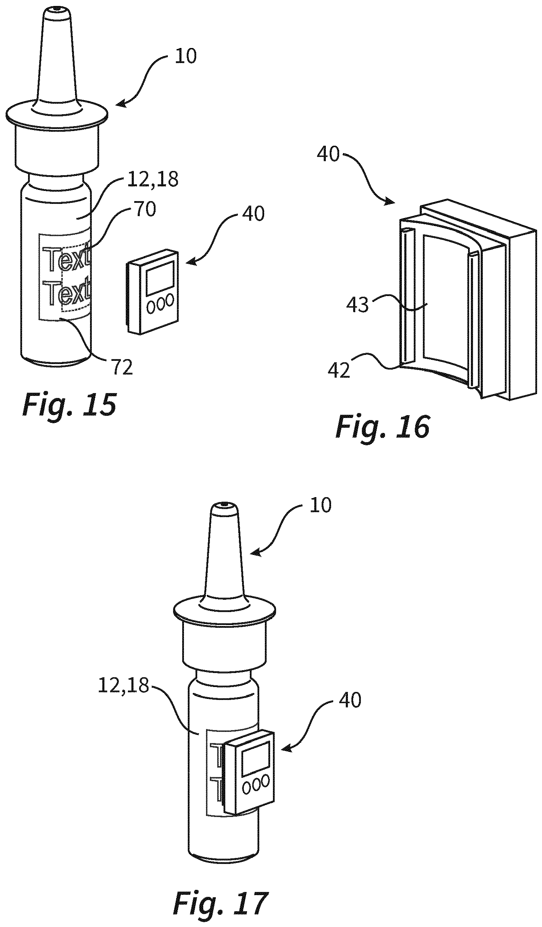

Die Fig. 15 bis 17 zeigen eine alternative Form der Befestigung der Auswerteeinheit 40 am Spendergehäuse 12. Hier ist vorgesehen, dass am Speicherkörper 18 des Spenders 10 ein Etikett 72 angebracht ist. In dieses Etikett 72 ist eine magnetisierbare Haltefläche 70 integriert, die beispielsweise aus einem magnetisierbaren Metall besteht.

Korrespondierend hierzu ist an der Anlageseite 42 derAuswerteeinheit 40 ein Permanentmagnet 43 vorgesehen. Wird die Auswerteeinheit 40 in den Bereich der Haltefläche 70 geführt, so wird sie magnetisch angezogen und verbindet sich somit magnetisch mit dem Speicherkörper 18, wie in Fig. 17 dargestellt ist. Der Permanentmagnet 43 ist vorzugsweise als Neodym-Magnet ausgebildet, um eine für einen sicheren Halt ausreichende Haltekraft zu verursachen.

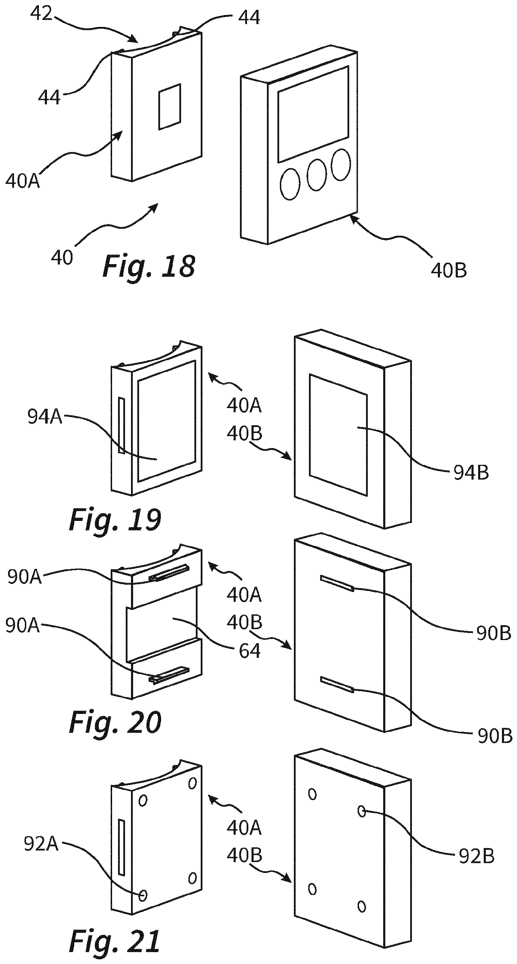

Die Fig. 18 bis 21 verdeutlichen die Möglichkeit, die Auswerteeinheit 40 modularzu gestalten. Wie anhand der Fig. 18 verdeutlicht, untergliedert sich die Auswerteeinheit 40 in solchen Fällen in eine Befestigungseinheit 40A, an der die Anlageseite 42 sowie die Anlagerippen 44 vorgesehen sind, sowie in eine Elektronikeinheit 40B, die die überwiegende Zahl an elektrischen/elektronischen Komponenten aufweist, vorzugsweise die Gesamtheit der elektronischen Komponenten. Es kann allerdings vorgesehen sein, dass die Befestigungseinheit 40A über ein elektronischen Identifikationsmittel wie einen RFID-Chip verfügt, damit die Elektronikeinheit 40B erkennen kann, ob sie mit einer Befestigungseinheit 40A verbunden ist bzw. mit welcher Befestigungseinheit 40A sie verbunden ist.

Die Teileinheiten 40A, 40B sind vorzugsweise werkzeuglos miteinander koppelbar. Bei der Gestaltung der Fig. 19 sind die beiden Teileinheiten mit magnetischen oder magnetisierbaren Kopplungsflächen 94A, 94B verbunden.

Bei der Gestaltung der Fig. 20 sind Rastlaschen 90A an der Befestigungseinheit 40A vorgesehen, die in Rastöffnungen 90B an der Elektronikeinheit 40B verrasten. Vorzugsweise sind die Rastlaschen 90A abweichend von der Gestaltung der Fig. 20 manuell auslenkbar, um die Verbindung wieder trennen zu können. Es kann jedoch auch vorgesehen sein, dass die Verbindung der Elektronikeinheit 40B mit der Befestigungseinheit 40A bestimmungsgemäß unlösbar ist, so dass eine entsprechende Kopplung nur einmalig möglich ist, insbesondere bei einer bereits im Zuge der Herstellung stattfindenden Festlegung auf eine bestimmte Befestigungseinheit 40A. Bei der Gestaltung der Fig. 20 ist darüber hinaus ersichtlich, dass an einer der Einheiten, vorliegend an der Befestigungseinheit 40A, eine Vertiefung vorgesehen ist, die der Bildung des Schachtes 64 und der Aufnahme eines Befestigungsbandes 60 dient.

Bei der Gestaltung der Fig. 21 sind Schraublöcher 92A, 92B an der Elektronikeinheit 40B und der Befestigungseinheit 40A vorgesehen, durch die hindurch die beiden Teileinheiten miteinander verschraubt werden können. Diese Art der Verbindung ist üblicherweise nicht dafür vorgesehen, vom Endanwender gelöst zu werden, sondern dient dem Zweck, im Zuge der Herstellung eine einheitliche Elektronikeinheit 40B dauerhaft mit einer Befestigungseinheit 40A verbinden zu können.

## Patentansprüche

1. Spender (10) zum Austrag pharmazeutischer oder kosmetischer Medien mit den folgenden Merkmalen:
a. der Spender (10) weist ein Gehäuse (12) auf, und
b. der Spender (10) weist einen Medienspeicher (14) auf, und
c. der Spender (10) weist eine Austragöffnung (16) auf, durch die Medium aus dem Medienspeicher (14) in eine umgebende Atmosphäre abgegeben werden kann, und
d. der Spender (10) weist eine elektronische Auswerteeinheit (40) zur Erfassung der Nutzung des Spenders auf, und
e. die elektronische Auswerteeinheit (40) ist zur lösbaren Anbringung am Gehäuse (12) des Spenders (10) ausgebildet,
**gekennzeichnet durch** die folgenden Merkmale:
f. die elektronische Auswerteeinheit (40) weist eine Anlageseite (42) auf, die im angebrachten Zustand der Auswerteeinheit (40) in Richtung des Gehäuses (12) weist, und
g. die elektronische Auswerteeinheit (40) weist an der Anlageseite (42) zwei zueinander parallele Anlagerippen (44) zur Anlage am Gehäuse (12) auf, insbesondere zur Anlage an einem Speicherkörper (18) des Medienspeichers (14).

2. Spender (10) nach Anspruch 1 mit mindestens einem der folgenden weiteren Merkmale:
a. die Anlagerippen (44) weisen einen Abstand (46A) voneinander von mindestens 8 mm auf, und/oder
b. die Anlagerippen (44) erheben sich gegenüber einem zwischen ihnen liegenden Zwischenbereich (48) um einen Abstand (46B) von mindestens 1,5 mm.

3. Spender (10) nach Anspruch 1 oder 2 mit dem folgenden weiteren Merkmal:
a. mindestens eine Anlagerippe (44) ist an einem elastischen Ausleger (50) angebracht.

4. Spender nach einem der vorstehenden Ansprüche mit dem folgenden weiteren Merkmal:
a. die Anlagerippen (44) werden durch mehrere zueinander fluchtende Rippensegmente gebildet, die vorzugsweise jeweils an einem elastischen Ausleger (50) vorgesehen sind.

5. Spender (10) nach einem der vorstehenden Ansprüche mit dem folgenden weiteren Merkmal:
a. die elektronische Auswerteeinheit (40) weist ein formflexibles Befestigungsband (60) auf, welches an der Auswerteeinheit (40) angebracht ist und das Gehäuse (12) umgibt, insbesondere den Speicherkörper (18).

6. Spender (10) nach Anspruch 5 mit dem folgenden weiteren Merkmal:
a. das Befestigungsband (60) ist als elastisches Band ausgebildet, insbesondere vorzugsweise mit mindestens einem der folgenden zusätzlichen Merkmale:
b. das Befestigungsband (60) ist als O-Ring (61) ausgebildet, und/oder
c. das Befestigungsband (60) ist als geschlossenes Band ausgebildet, welches in Form eines Doppelbandes das Gehäuse umgibt und im Bereich zweier umlenkender Schlaufen (60A) an einer Haupteinheit der Auswerteeinheit befestigt ist, oder
d. das Befestigungsband (60) ist als geschlossenes Band ausgebildet, welches das Gehäuse vollständig umgebend an diesem angebracht ist.

7. Spender (10) nach Anspruch 5 oder 6 mit den folgenden weiteren Merkmalen:
a. das Befestigungsband (60) umfasst zwei Bandabschnitte (60B, 60C), die jeweils an einer Haupteinheit der Auswerteeinheit (40) befestigt sind und die miteinander im Bereich eines Verschlusses (62) verbunden sind, oder
b. das Befestigungsband (60) ist als längenveränderliches Band ausgebildet, welches mittels eines Verschlusses (62) in einer gewünschten Längenkonfiguration festgestellt werden kann,
vorzugsweise mit einem der folgenden zusätzlichen Merkmale:
c. der Verschluss (62) ist als Klettverschluss ausgebildet, und/oder
d. der Verschluss weist zusammenwirkende Verschlusselemente an beiden Bandabschnitten auf, wobei an einem der Bandabschnitte vorzugsweise eine Mehrzahl von Verschlusselementen vorgesehen sind, und/oder
e. der Verschluss (62) ist als magnetischer Verschluss ausgebildet.

8. Spender nach einem der vorstehenden Ansprüche mit den folgenden weiteren Merkmalen:
a. an der Außenseite des Gehäuses (12), insbesondere an einem Speicherkörper (18) des Gehäuses (12), ist eine magnetische oder magnetisierbare Haltefläche (70) vorgesehen, und
b. eine Anlageseite (42) der Auswerteeinheit (40) ist hierzu korrespondierend magnetisch oder magnetisierbar ausgebildet, so dass die Auswerteeinheit (40) magnetisch am Gehäuse (12) befestigt werden kann.

9. Spender (10) nach Anspruch 8 mit mindestens einem der folgenden weiteren Merkmale:
a. die magnetische oder magnetisierbare Haltefläche (70) ist durch ein beschriftetes Etikett (72) überdeckt oder in ein beschriftetes Etikett integriert und/oder
b. die magnetische oder magnetisierbare Haltefläche wird durch eine am Gehäuse klemmend befestigte Haltehülse oder Halteklemme gebildet und/oder
c. die magnetische oder magnetisierbare Haltefläche (70) ist klebend am Gehäuse (12) befestigt.

10. Spender nach einem der vorstehenden Ansprüche mit den folgenden weiteren Merkmalen:
a. die Auswerteeinheit weist mindestens zwei und vorzugsweise mindestens drei elastisch auslenkbare Haltearme auf, die das Gehäuse, insbesondere einen Speicherkörper des Gehäuses, umgreifen und damit einen klemmenden Halt der Auswerteinheit am Gehäuse bewirken,
vorzugsweise mit dem folgenden zusätzlichen Merkmal:
b. es sind mindestens drei Haltearme vorgesehen, von denen zwei Haltearme sich in einer ersten Erstreckungsrichtung erstrecken und von denen der dritte Haltearm zwischen diesen beiden Haltearmen angeordnet ist und sich in entgegengesetzter Erstreckungsrichtung erstreckt.

11. Spender (10) nach einem der vorstehenden Ansprüche mit den folgenden weiteren Merkmalen:
a. dieAuswerteeinheit (40) umfassteine Befestigungseinheit (40A) und eine Elektronikeinheit (40B), und
b. die Befestigungseinheit (40A) ist zur lösbaren Anbringung am Gehäuse (12) ausgebildet, und
c. die Elektronikeinheit (40B) ist zur lösbaren Anbringung an der Befestigungseinheit (40A) vorgesehen.

12. Spender (10) nach Anspruch 11 mit mindestens einem der folgenden zusätzlichen Merkmale:
a. die Befestigungseinheit (40A) ist zur werkzeuglos lösbaren Anbringung am Gehäuse (12) ausgebildet, und/oder
b. die Elektronikeinheit (40B) ist magnetisch an der Befestigungseinheit (40A) befestigt, und/oder
c. die Elektronikeinheit (40B) ist durch einen Rastmechanismus mit einer elastisch auslenkbaren Rastlasche (90) an der Befestigungseinheit (40A) befestigt, und/oder
d. die Befestigungseinheit (40A) weist ein Identifikationsmerkmal auf, insbesondere abgelegt in einem RFID-Chip der Befestigungseinheit, welcher durch einen Sensor (26B) der Elektronikeinheit (40B) auslesbar ist.

13. Spender (10) nach einem der vorstehenden Ansprüche mit mindestens einem der folgenden weiteren Merkmale:
a. die Auswerteeinheit (40) umfasst mindestens einen Sensor (26A, 26B, 26C), insbesondere einen Bewegungssensor (26A) und/oder ein Mikrofon (26C), und/oder
b. die Auswerteeinheit (40) umfasst eine Ausgabeeinrichtung (24), insbesondere in Form mindestens einer LED, einer Anzeigeeinrichtung (24), eines Vibrators und/oder eines Lautsprechers,
c. die Auswerteeinheit umfasst ein Funkmodul (22), insbesondere ein Bluetooth- und/oder WIFI-Funkmodul (22), und/oder
d. die Auswerteeinheit umfasst eine Energiequelle (25), insbesondere in Form einer Batterie.

14. Spender (10) nach einem der vorstehenden Ansprüche mit dem folgenden weiteren Merkmal:
a. der Spender (10) ist als Flüssigkeitsspender (10) ausgebildet, wobei der Medienspeicher (14) als Flüssigkeitsspeicher (14) ausgebildet ist,
insbesondere mit mindestens einem der folgenden zusätzlichen Merkmale:
b. der Spender ist als Quetschflaschenspender ausgebildet und weist einen elastisch verformbaren Speicherkörper des Flüssigkeitsspeichers auf, durch dessen manuelle Komprimierung Flüssigkeit ausgetragen werden kann, oder
c. der Spender (10) istals Pumpspenderausgebildet, der über eine manuell betätigbare Pumpeinrichtung (30) verfügt, die Flüssigkeit aus dem Flüssigkeitsspeicher (14) zur Austragöffnung (16) fördert, und/oder
d. der Spender (10) ist als Sprühspender ausgebildet, und/oder
e. der Spender ist als Inhalator ausgebildet, insbesondere als Spender mit einem zur Abgabe einer Dosis der Flüssigkeit niederdrückbarem Container,
f. der Spender ist als Tropfenspender ausgebildet, insbesondere mit einer die Austragöffnung umgebenden Tropfenbildungsfläche, und/oder
g. der Spender ist als Nasalspender ausgebildet, insbesondere mit einem langgestreckten Nasalapplikator, an dessen distalem Ende die Austragöffnung vorgesehen ist, und/oder
h. der Medienspeicher (14) des Spenders weist ein Volumen von maximal 200 ml auf, vorzugsweise von maximal 100 ml, und/oder
i. der Medienspeicher (14) des Spenders ist mit einer pharmazeutischen Flüssigkeit befüllt.

15. Auswerteeinheit füreinen Spender nach einem dervorstehenden Ansprüche mit den folgenden Merkmalen:
a. die Auswerteeinheit (40) ist zur lösbaren Anbringung an einem Spender (10) ausgebildet, und
b. die elektronische Auswerteeinheit (40) ist zur Erfassung der Nutzung des Spenders ausgebildet und weist hierfür mindestens einen Sensor (26A, 26C) auf,
**gekennzeichnet durch** das folgende weitere Merkmal:
c. die elektronische Auswerteeinheit (40) weist eine Anlageseite (42) auf, die im angebrachten Zustand der Auswerteeinheit (40) in Richtung des Gehäuses (12) weist, wobei an der Anlageseite (42) zwei zueinander parallele Anlagerippen (44) zur Anlage am Gehäuse (12) vorgesehen sind,
vorzugsweise mit mindestens einem der folgenden zusätzlichen Merkmale:
d. die elektronische Auswerteeinheit (40) umfasst eine Mehrzahl von Befestigungsbändern, die wahlfrei an der Auswerteinheit (40), insbesondere an der Befestigungseinheit (40A) anbringbar sind, und/oder
e. die Auswerteeinheit (40) umfasst eine Mehrzahl von Befestigungseinheiten (40A), die wahlfrei an der Elektronikeinheit (40B) angebracht werden können.
